# EUROPEAN PATENT APPLICATION

(11) **EP 4 342 482 A1**
(43) Date of publication of application: **27.03.2024**
(21) Application number: 22197387.8
(22) Date of filing: 23.09.2022
(51) Int. Cl.: A61K 36/58, A61P 17/00, A61P 17/06, A61P 35/00, A61K 8/00

(54) **NEEM TREE LEAVES EXTRACT**

(71) Applicant: Vorderwülbecke, Laurenz, 80469 München (DE)
(72) Inventor: Vorderwülbecke, Laurenz, 80469 München (DE)
(74) Representative: Weickmann & Weickmann PartmbB

(57) **Abstract**

A Neem tree leaf extract of the present invention is enriched in Nimbolide and/or deplete in Azadirachtin content compare dot the relative average proportional content of these components in Neem tree leaves. A method for producing such inventive extract comprises applying supercritical CO₂ extraction at conditions favoring selective enrichment of the Nimbolide content and/or selective depletion in Azadirachtine content in the extract.

## Description

The present invention relates to an extract of Neem tree *(Azadirachta indica)* leaves, which extract is enriched in certain ingredients of Neem tree leaves and/or depleted in others. Furthermore, the invention relates to methods of obtaining the inventive extract as well as to cosmetic and pharmaceutical compositions comprising such extract. The invention also relates to inventive Neem leaf extracts for use in skin care and dermatology as well as in other medical fields, in which the extracts can be used for treating and alleviating diseases and their symptoms.

### Background and prior art

For thousands of years, the Neem tree *(Azadirachta indica)* has been known as a medical plant. It is often described and considered as the "village pharmacy", especially in rural areas in India, however, also widely used in other parts of the world. Plant parts, like leaves, bark and seeds as well as products obtained therefrom, are used in all kinds of application forms and are also established as an Ayurveda medicinal reservoir. Many of the uses of Neem tree products are based on traditional knowledge of people in countries, in which Neem trees have been growing for centuries.

The various plant parts are considered effective for different, but partly also for the same or, at least, overlapping purposes. For example, Neem oil obtained from the seeds of the Neem tree is well-known for its use as pesticide but also for cosmetic use and purposes as well as a natural spermicide. Oral uptake, on the other hand, is not recommended for Neem oil. Neem tree leaves are widely used in the cosmetic industry but also in medicinal applications.

In the Western hemisphere, scientific research was initiated by the work of Prof. Schmutterer (e.g., summarized in: Heinrich Schmutterer (Hrsg.): The neem tree Azadirachta indica (A. Juss.) and other meliaceous plants. Sources of unique natural products for integrated pest management, medicine, industry and other purposes. VCH Verlagsgesellschaft, Weinheim 1995, ISBN 3-527-30054-6), who investigated the use of Neem tree products as pesticides after he discovered Neem trees as the only surviving plants after a particularly gruesome locust plague. His research mainly focused on Azadirachtin and other plant ingredients which show a pesticidal effect.

Truly scientific literature regarding ingredients, isolation of such ingredients and uses or application forms exists only to a very limited extent and is not conclusive. Studies reflected in such literature often do not follow scientific standards and results described therein often turn out to be not reproducible. Often, there is also a lack of reliable analysis for comparing observed effects. For example, the provenance of the Neem leaves is often stated as *"from our university garden"* and the actual content of ingredients in this source material is not analyzed at all, or at least not in a reproducible manner.

In addition to its use in traditional medicine, Neem as well as certain specific components, which can be enriched or isolated from the plant have also been investigated more recently for the treatment of various diseases, especially for application in cancer therapies. Studies have shown surprising effects for the application of such substances and it was assumed that anti-cancer effects are caused by a modulation of multiple cellular processes upon application of Neem extracts or purified or isolated Neem components.

One of these components of interest is Nimbolide for which an activity has been shown against a number of different types of cancer in *in vivo* and *in vitro* studies. It has also been shown that Nimbolide is effective in inducing apoptosis while inhibiting metastasis and angiogenesis. Further components of the Neem tree have been shown to possess an anti-inflammatory effect and other medically relevant properties.

The prior art describes various methods for obtaining extracts including the various Neem ingredients. E.g., US 2020/0069636 A1 summarizes some relevant prior art documents and methods described therein, which include extraction methods using specific solvents and solvent combinations and partly also precipitation and chromatographic fractionation methods. The relevant documents include WO 2015/035199 A1, CN 101972246 B, CN 103864876 A, US 5,370,873 A, JP 2009/274956 A and WO 2007/137389 A1. However, these methods are considered as unsuitable for their use of relatively high temperatures for the separation, which is considered to destroy or modify the molecular structure of temperature-sensitive components with therapeutic value. Also, using solvents is considered as undesirable as these can have adverse effects on the health of humans and even can be carcinogenic themselves.

The authors of US 2020/0069636 A1 propose a process for preparing a CO₂ extract of Neem in which heat-sensitive phytoconstituents are obtained during the extraction process by applying supercritical CO₂ extraction conditions at a temperature of less than 45°C which has been known to not negatively affect heat-sensitive constituents. The method applied, however, requires a number of process steps including repeated extractions: More specifically, in addition to a supercritical CO₂ extraction, also a further extraction using a mixture of CO₂ and ethyl alcohol is performed and, in order to remove the solvent, a vacuum distillation is required in this method. Thus, the process disclosed in this document requires quite high procedural efforts and still cannot completely eliminate the use of a solvent in order to achieve a satisfactory yield of the desired Neem components.

Within the context of the research into a medical use of Nimbolide for various diseases and medical conditions, also the use of commercially available Nimbolide can be contemplated. However, especially when diseases are concerned, which while not being life-threatening nevertheless require alleviation of symptoms and treatment, use of such commercially available Nimbolide is not a preferable option due to the very high costs for even very small amounts of this substance. Furthermore, synergistic effects provided by the presence of other Neem components are lost in such case.

For many applications and especially for certain medical applications, several Neem components like Azadirachtin are considered undesirable as they can also have a negative effect on humans when applied or ingested, especially when applied over a longer period of time. Also, for dermatological purposes, the inclusion of such substances is undesirable. Thus, the objects underlying the present invention are, on the one hand, the provision of a Neem extract containing an elevated concentration of Nimbolide compared to known extracts, and the provision of a simple and cost-effective method for producing such extract while avoiding inclusion of undesirable substances from the Neem plant as well as from the extraction process.

### Brief description of the invention

The object has been solved by, as a first aspect of the invention, providing an extract of Neem tree *(Azadirachta indica)* leaves, which extract is enriched in Nimbolide and/or depleted in Azadirachtin content compared to the relative average proportional content of these components in Neem tree leaves.

In a second aspect of the invention a method for producing the inventive extract of Neem tree leaves is provided, which method comprises applying supercritical CO₂ extraction at conditions favoring selective enrichment of the Nimbolide content and/or selective depletion in Azadirachtin content in the extract compared to the average proportional content of these components on Neem tree leaves.

In a third and fourth aspect of the invention, a cosmetic composition as well as a pharmaceutical composition are provided comprising an inventive extract as defined above. In a fourth aspect of the invention, the inventive extract as well as the cosmetic or pharmaceutical compositions of the present invention are provided for use in dermatology, treatment of cancer diseases and treatment of inflammatory conditions.

### Detailed description of the invention

Within the context of their research, the current inventors were able to establish a method, which allows for extraction of Nimbolide and some further ingredients of Neem tree leaves in a highly efficient and selective manner.

For example, the Nimbolide content in inventive Neem extracts can be greatly enhanced compared to extracts obtained by previously described extraction methods. More specifically, a Nimbolide content in the inventive extract of 4-25% by weight can be obtained, which is much higher than the respective contents obtained in the prior art. In preferred embodiments, the Nimbolide content in an extract of the present invention is at least 5% by weight, more preferably more than 7% by weight. In especially preferred embodiments, the invention achieves an Nimbolide content in the extract of 10% by weight or more. According to the invention, the content of Azadirachtin A and B in the extract should be less than 1% by weight and preferably less than 0.15% by weight. Other ingredients like Nimbin can also be present, usually in a relative amount of 0.1-0.6% by weight, whereas most preferably the inventive extract does not contain detectable levels of Azadirachtin A and B, at all. Depending on the actual extraction conditions, the inventive extracts can also include contents of substances selected from polyphenols, tannins and catechins, e.g., in an amount of 20-30% by weight, flavonoids, e.g., at 20-32% by weight, terpenoids, e.g., at 10-20% by weight, pigments, e.g., at 15-25% by weight, and residual hydrocarbons being present, e.g., at 10-20% by weight.

The inventive Neem leaf extracts are obtainable by the inventive method as described in the following.

The inventive method of producing an extract of Neem tree leaves as defined above, comprises applying supercritical CO₂ extraction at conditions favoring selective enrichment of the Nimbolide content and/or selective depletion in Azadirachtin content in the extract compared to the average proportional content of these components in Neem tree leaves.

According to preferred embodiments of the present invention, the method can also include the selective removal of one or more extract components other than Nimbolide, thus, again increasing the relative content of Nimbolide in the extract. In this context, e.g., substances responsible for a mossy smell and/or a greenish color of the extract can be removed by such additional selective removal performed by any technique which is applicable in this context. Such techniques are well-known to the skilled person and include further extraction methods and chromatographic methods like, e.g., flash chromatography.

The inventive method comprises harvesting of Neem tree leaves or the provision of Neem tree leaves in an appropriate amount. Preferably, the Neem tree leaves have not been treated with pesticides before or after being harvested and leaves from organic farming are preferred sources for the starting material of the inventive method. Neem tree leaves are washed and dried and subsequently crushed or ground before they are subjected to supercritical CO₂ extraction. The intensity of the crushing or grounding can be adapted as required and appropriate for the extraction plant and can e.g., be varied to obtain crushed leaves with approximately the size of rolled oats, but also more finely ground and pulverized material can be used.

In preferred embodiments of the present invention, the leaves are dried at a temperature of up to 45°C, and a preferred method for drying includes use of an air-pressure apparatus.

The supercritical CO₂ extraction is preferably performed at a pressure of 200-600 bar, more specifically at 250-350 bar. The preferred temperature for the supercritical CO₂ extraction is 50-100°C and more specifically 55-85°C and especially 60-80°C.

The inventors detected that in spite of indications provided in prior art documents and adhered to by other groups for their extraction methods (e.g., US 2020/0069636 A1) that the integrity of phytoconstituents would be compromised by using temperatures above 45°C, application of relatively high temperatures in the extraction process leads to an unexpected increase of the Nimbolide content in the inventive extracts. In contrast to the prior art, in which temperatures of lower than 45°C were applied, the present invention uses considerably elevated temperatures which results in exceptionally high yields of unimpaired Nimbolide and a complete or nearly complete elimination of Azadirachtin.

The conditions of the inventive method further comprise the use of 5 to 30 kg CO₂/kg*h crushed or ground Neem tree leaves, preferably 10 kg CO₂/kg*h Neem tree leaves. According to preferred embodiments of the invention, the supercritical CO₂ extraction is performed over a period of 1 to 5 hours, preferably 2 to 4 hours and most preferably 2,5 to 3,5 hours. Further preferably, during the overall extraction process, 20 to 40 kg CO₂/kg Neem tree leaves are used for extraction, more preferably 25 to 35 kg CO₂/kg Neem tree leaves and most preferred 30 kg CO₂/kg Neem tree leaves.

The inventive method produces highly purified Neem extracts containing high contents of Nimbolide and results in very low contents of Azadirachtin or the complete elimination thereof by applying supercritical CO₂ extraction only, and thus allows for avoiding any other solvent to be included in the extract. One of the advantages of the inventive method is that, due to the applied extraction method, the obtained extracts are free from extractants like alcohols which are most commonly used in prior art methods. For many applications, however, it is preferable or even essential that no alcohols are present in the extracts and products obtained therefrom, like in dermatological applications in which the presence of alcohols can lead to skin dryness or other adverse skin reactions.

Furthermore, the inventive method does not require any pretreatment of the dried and crushed or shredded leave material as described in the prior art.

After performing the extraction, it is possible and preferred to warm up the obtained extract to a temperature of about 40°C to about 60°C and to perform a mixing step in order to improve the homogeneity of the obtained extract.

The inventive method for the first time provides a one-step extraction process of crushed Neem tree leaves, which leads to a highly satisfactory yield of Nimbolide in the obtained extract. The extract can be directly used in all known and conceivable applications of Neem extract and especially of Nimbolide. The extract has also been shown to be very well tolerable in any application form and formulation.

Further subjects of the present invention are cosmetic and pharmaceutical compositions comprising the inventive extract or extract obtained by the above-described inventive method, respectively.

A cosmetic composition of the present invention preferably contains an inventive Neem leaf extract in a proportion of 0.1-5% by weight, preferably 0.25 to 3 wt-%, and depending on the exact application form and intended indication of the cosmetic composition, the amount of extract contained in the cosmetic composition can also be increased or decreased.

A cosmetic composition of the present invention further comprises a cosmetic base. Such cosmetic base can include water-in-oil emulsions, oil-in-water emulsions, e.g., a gel base, an ointment/creme base, a lotion base, or any other form of cosmetic base. The inventive extract is readily soluble in oil, however not soluble in water. Addition of appropriate emulsifiers, however, allows for use of the inventive extracts also in water-based application forms.

Depending on the intended treatment, the inventive cosmetic composition can also include other dermatologically active substances and/or e.g., analgesics.

For the inventive pharmaceutical composition, the content of the inventive extract within the composition is not restricted. The pharmaceutical composition usually contains auxiliary materials/excipients and/or carriers and possibly also further additives for the intended application and formulation. The pharmaceutically active constituent within the composition can be 100% inventive Neem extract or a combination with other pharmaceutically active substances, depending on the intended medical indication. E.g., for uses in dermatology, also the pharmaceutical composition preferably contains an appropriate base for dermal application, for other medical applications the extract can be used either in pure form or be combined with required or useful further substances. For certain applications also the formulation as tablets, capsules, lozenges, or liquid forms for topic or oral administration or for injection are further exemplary but non limiting dosage forms.

Applicable uses of the inventive Neem leaf extracts include all medical applications for Neem products and especially for Nimbolide which have already been disclosed in the prior art.

Another subject matter of the present invention, accordingly, is an extract of the present invention or a pharmaceutical composition as described above for use in medicine, preferably for dermatology, especially the treatment of psoriasis, neurodermitis, eczema and acne, for treatment of cancer diseases and for treatment of (other) inflammatory conditions.

The following examples and figures further illustrate the invention:
Fig. 1 shows the reduction of oxidative stress as a result of application of different concentrations of the inventive Neem leaf extract in a skin model study compared to a positive control and other active substances.
Fig. 2 shows a further skin model experiment and the anti-inflammatory effect of the inventive Neem leaf extract in different concentrations compared to an untreated control.

### Examples

### Example 1: Preparation and analysis of a Neem tree leaf extract

Neem tree leaves were harvested, washed, and dried at a temperature of 45°C at most via air pressure drying. After the drying process, the leaves were transferred to a hammer mill and ground to a powder.

Supercritical CO₂ extraction was performed at 300 bar and 80°C for 3 hours at 30m.rel (kg CO₂/kg leaf powder). No further extractants or pretreatments were applied. The obtained extract was heated to 40°C and mixed to achieve homogeneity.

The raw extract was standardized with caprylyl glycol, wherein roughly a ratio of 60% extract to 40% glycol was used for the last standardization.

The following table 1 shows the HPLC analysis of several extract batches:

**Table 1:**

| Extraction | Description | Aza A | Aza B | Nimbolide | Nimbin |
|---|---|---|---|---|---|
| Sample extraction #1 | | BDL | BDL | 18,64% | 0,18% |
| Sample extraction #2 | Older, less potent leaves as source material | BDL | BDL | 6,43% | 0,36% |
| Sample extraction #3 | Older, less potent leaves as source material | BDL | BDL | 9,88% | 0,24% |
| Sample extraction #4 | | BDL | BDL | 15,47% | 0,45% |

### Example 2: In vitro study on a dermatological model

An *in vitro* study was performed on 3D skin models to assess the anti-oxidative potential and the anti-inflammatory properties of the inventive Neem tree leaf extract and skin barrier regeneration following application of the extract.

In the tests performed, Neem leaf extract was used in concentrations of 0,5% to 2,5% and compared to a positive control of untreated skin model and 2,5% Vitamin C. A further comparison was made to application of 0,01% Astaxanthin which is commonly used as the "gold standard" for anti-oxidative substances.

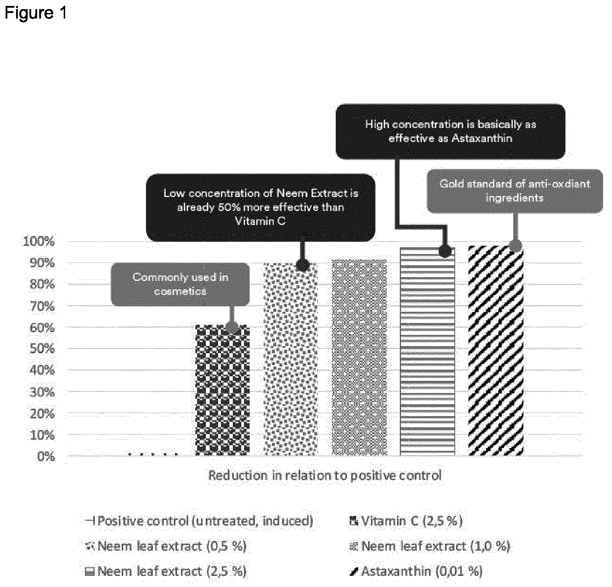

The study reveals a significant reduction of oxidative cell stress. Even the smallest doses of extract of 0,5% showed a remarkable improvement over Vitamin C application and reduced cell stress by more than 90%. The 2,5% concentration of extract had about the same effect as Astaxanthin on cell stress reduction (Fig. 1).

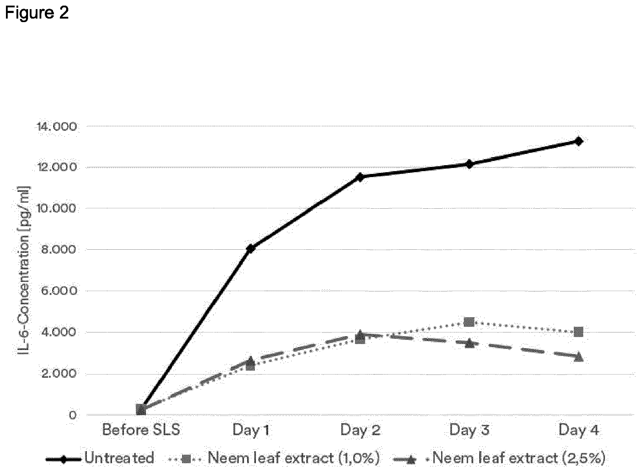

Furthermore, the study shows a pronounced anti-inflammatory effect of the inventive extract. The skin model was treated with SLS on day 1. Directly following this SLS treatment, the inventive extract was applied to the skin model. The extract-treated skin model showed markedly lower IL-6 concentrations than untreated samples. Already on day 3, a further distinct decrease of the IL-6 concentration was detected when using 2,5% extract. Treatment with a 1% extract showed a corresponding decrease at day 4 (Fig. 2).

The skin model studies clearly show a significant beneficial effect of Neem leaf extracts of the present invention even at low concentrations for both its anti-oxidative and anti-inflammatory properties.

## Claims

1. An extract of Neem tree (Azadirachta indica) leaves which extract is enriched in Nimbolide and/or depleted in Azadirachtin content compared to the relative average proportional content of these components in Neem tree leaves.

2. The extract of Neem tree leaves of claim 1, which contains 4 to 25 % by weight Nimbolide, preferably at least 5% by weight and more preferably 10 to 20% by weight Nimbolide.

3. The extract of Neem tree leaves of claims 1 or 2, which contains less than 1% by weight, preferably less than 0.15% by weight Azadirachtines.

4. A method of producing an extract of Neem tree leaves according to anyone of claims 1 to 3, wherein the method comprises applying supercritical CO₂ extraction at conditions favoring selective enrichment of the Nimbolide content and/or selective depletion in Azadirachtine content in the extract compared to the average proportional content of these components in Neem tree leaves.

5. The method of claim 4, wherein proportional content of Nimbolide in the extract obtained by supercritical CO₂ extraction is further increased by selective removal of one or more other extract components.

6. The method of claims 4 or 5, wherein Neem tree leaves are harvested, washed, dried and crushed or ground prior to supercritical CO₂ extraction.

7. The method of claim 6, wherein drying is performed at a temperature of up to 45 °C, preferably via air pressure drying.

8. The method of anyone of claims 4 to 7, wherein the supercritical CO₂ extraction is performed at a pressure of 200 to 600 bar, preferably 250 to350 bar, and/or at a temperature of 50 to 100°C, preferably 55 to 85 °C, and most preferably 60 to 80 °C.

9. The method of anyone of claims 4 to 8, wherein the supercritical CO₂ extraction is performed for 1 to 5 hours, preferably 2 to 4 hours, and most preferably 2.5 to 3.5 hours.

10. The method according to anyone of claims 4 to 10, wherein the supercritical CO₂ extraction is performed using 20 to 40 kg CO₂ per kg crushed or ground Neem tree leaves, preferably 25 to 35 kg CO₂ per kg, and/or wherein the supercritical CO₂ extraction is performed using about 10 kg CO₂ per kg Neem tree leaves and per hour extraction time.

11. A cosmetic composition comprising an extract of anyone of claims 1 to 3 or an extract obtained by a method of anyone of claims 4 to 10.

12. The cosmetic of claim 12, in which the extract is contained in an amount of 0.1 to 5 wt.-%, preferably 0.25 to 3 wt.-% based on the total weight of the composition.

13. A pharmaceutical composition comprising an extract of anyone of claims 1 to 4 or an extract obtained by a method of anyone of claims 4 to 10.

14. An extract of anyone of claims 1 to 3 or a cosmetic or pharmaceutical composition according to anyone of claims 11 to 13 for use in dermatology, especially treatment of psoriasis, for treatment of cancer diseases and for treatment of inflammatory conditions.
